(19) Europäisches Patentamt
European Patent Office
Office européen des brevets

(11) EP 3 010 513 B1

(12) **EUROPEAN PATENT SPECIFICATION**

(45) Date of publication and mention of the grant of the patent:
**05.06.2019 Bulletin 2019/23**

(21) Application number: **14732136.8**

(22) Date of filing: **16.06.2014**

(51) Int Cl.:
*A61K 31/7068* (2006.01) *A61K 31/40* (2006.01)
*A61P 35/00* (2006.01)

(86) International application number:
**PCT/EP2014/062490**

(87) International publication number:
**WO 2014/202492 (24.12.2014 Gazette 2014/52)**

(54) **COMBINATION OF RO5503781 AND CAPECITABINE FOR CANCER THERAPY**

KOMBINATION AUS RO5503781 UND CAPECITABIN FÜR KREBSTHERAPIE

ASSOCIATION DE RO5503781 ET DE CAPÉCITABINE POUR UNE CANCÉROTHÉRAPIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2013 US 201361836894 P**

(43) Date of publication of application:
**27.04.2016 Bulletin 2016/17**

(73) Proprietor: **F. Hoffmann-La Roche AG**
**4070 Basel (CH)**

(72) Inventors:
- **HIGGINS, Brian**
**Fresh Meadows, New York 11365 (US)**
- **NICHOLS, Gwen**
**New York, New York 10128 (US)**
- **PACKMAN, Kathryn E.**
**Newton, Massachusetts 02461 (US)**

(74) Representative: **Beyermann, Jochen Carl**
**F. Hoffmann-La Roche AG**
**CLP - Patent Department**
**Grenzacherstrasse 124**
**4070 Basel (CH)**

(56) References cited:
**WO-A2-2008/034039**

- **KRZYSZTOF ZAK ET AL: "Mdm2 and MdmX inhibitors for the treatment of cancer: a patent review (2011 - present)", EXPERT OPINION ON THERAPEUTIC PATENTS, vol. 23, no. 4, 1 April 2013 (2013-04-01), pages 425-448, XP055107012, ISSN: 1354-3776, DOI: 10.1517/13543776.2013.765405**
- **Asfar S. Azmi ET AL: "Network Perspectives on HDM2 Inhibitor Chemotherapy Combinations", Current Pharmaceutical Design, 1 February 2011 (2011-02-01), pages 640-652, XP055137179, Netherlands Retrieved from the Internet: URL:http://www.eurekaselect.com/openurl/content.php?genre=article&issn=13816128&volume=17&issue=6&spage=640**
- **QINGJIE DING ET AL: "Discovery of RG7388, a Potent and Selective p53-MDM2 Inhibitor in Clinical Development", JOURNAL OF MEDICINAL CHEMISTRY, vol. 56, no. 14, 25 July 2013 (2013-07-25) , pages 5979-5983, XP055109745, ISSN: 0022-2623, DOI: 10.1021/jm400487c**
- **TONGSEN ZHENG ET AL: "Disruption of p73-MDM2 binding synergizes with gemcitabine to induce apoptosis in HuCCT1 cholangiocarcinoma cell line with p53 mutation", TUMOR BIOLOGY, vol. 31, no. 4, 27 April 2010 (2010-04-27) , pages 287-295, XP055137552, ISSN: 1010-4283, DOI: 10.1007/s13277-010-0035-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 010 513 B1

## Description

## FIELD OF THE INVENTION

The present invention is directed to a method of cancer therapy by administering (i) a pharmaceutical composition containing (i) a compound of formula

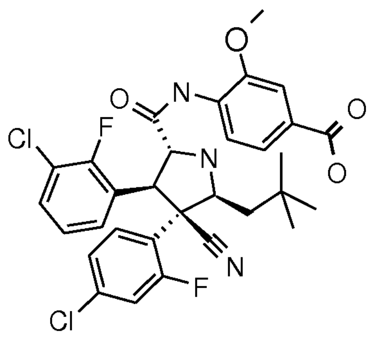

(A)

4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid (Compound A) an antagonist of the p53/MDM2 interaction and (ii) a pharmaceutical composition containing capecitabine which is a fluoropyrimidine carbamate with antineoplastic activity. It is an orally administered systemic prodrug of 5'-doexy-5-fluorouridine (5'-DFUR) which is converted to 5-fluorouracil intracellularly, an antineoplastic agent. Capecitabine is marketed in the United States by Roche Laboratories under the brand name Xeloda®. The chemical name for capecitabine is 5'-deoxy-5-fluoro-N-[(pentyloxy)-carbonyl]-cytidine and has the following structural formula:

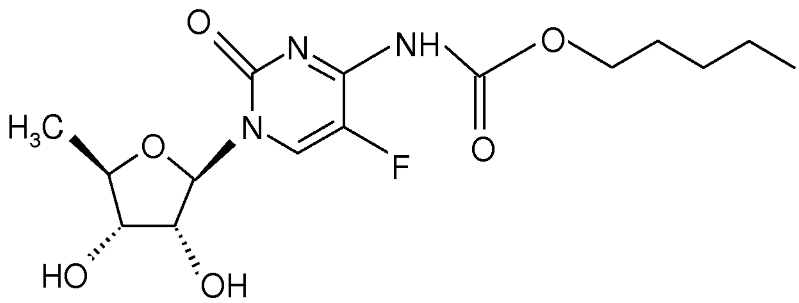

Capecitabine is covered in US patents, including US Pat. No. 4,966,891 and 5,472,949. Improved methods for the manufacture of capecitabine are also taught by US Pat. No. 5,453,497 and 5,476,932, and application USSN 60/532,266, filed December 22, 2003. Compound A is disclosed in WO2011/098398 and US Patent No. 8,354,444. Specific pharmaceutical preparations comprising Compound A are also disclosed in International Patent Application No. PCT/EP2014/050974. The invention is also directed to a kit containing both of the above compositions.

## BACKGROUND OF THE INVENTION

p53 is a transcription factor that can activate a panel of genes implicated in the regulation of cell cycle and apoptosis. p53 is a potent cell cycle inhibitor which is tightly regulated by MDM2 at the cellular level. MDM2 and p53 form a feedback control loop. MDM2 can bind p53 and inhibit its ability to transactivate p53-regulated genes. In addition, MDM2 mediates the ubiquitin-dependent degradation of p53. p53 can activate the expression of the MDM2 gene, thus raising the cellular level of MDM2 protein. This feedback control loop insures that both MDM2 and p53 are kept at a low level in normal proliferating cells. MDM2 is also a cofactor for E2F, which plays a central role in cell cycle regulation.

The ratio of MDM2 to p53 (E2F) is dysregulated in many cancers. Frequently occurring molecular defects in the p16INK4/p19ARF locus, for instance, have been shown to affect MDM2 protein degradation. Inhibition of MDM2-p53 interaction in tumor cells with activation of the p53-MDM2 pathway should lead to accumulation of p53, cell cycle arrest and/or apoptosis. The feasibility of p53/MDM2 antagonism as a strategy has been shown by the use of different macromolecular tools for inhibition of MDM2-p53 interaction (e.g. small molecules, antisense oligonucleotides, peptides).

Combinations of MDM2 inhibitors with chemotherapeutic agents have already been disclosed in the prior art (WO2008/034039; Curr. Pharm. Design, 2011, Vol. 17(6), 640-652; Tumor Biol. (2010) 31:287-295).

## DETAILED DESCRIPTION OF THE INVENTION

The present invention relates to a method of treating a patient suffering with cancer comprising administering to the patient, either concomitantly or sequentially, a first component consisting of a pharmaceutical composition containing as an active ingredient a therapeutically effective amount of a compound of 4-{ [(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid (Compound A) or a pharmaceutically acceptable salt or ester of said compound and a second component consisting of a pharmaceutical composition containing a therapeutically effective amount of capecitabine.

This combination of chemotherapeutic compounds is particularly useful in the treatment of colon cancer.

It was unexpectedly found that administration of the two components in accordance with the present invention results in improved antineoplastic effects that are significantly superior to the results obtained with each compound alone. Namely, administration of the two components in accordance with the present invention resulted in an improved therapeutic index (that is, superior efficacy) in comparison to either component alone without a significant increase in toxicity. Alternatively the invention permits reduction of the amount of at least one component (in comparison the amount typically given in monotherapy) while retaining a desirable therapeutic index. In preferred embodiments, the amount of both components (in comparison the amount typically given in monotherapy) is reduced affording reduced toxicity while still retaining a desirable therapeutic index.

In one embodiment, the present invention relates to a pharmaceutical product comprising a) a first component comprising Compound A; and b) a second component comprising capecitabine for the simultaneous or sequential treatment of cancer, in particular solid and/or hematological tumors such as breast, colon, colorectal, lung and pancreatic tumors, sarcoma or leukemias such as acute myelocytic leukemia (AML).

In another embodiment there is provided Compound A and capecitabine for the simultaneous or sequential use in the treatment of cancer, in particular solid and/or hematological tumors such as breast, colon, colorectal, lung and pancreatic tumors, sarcoma or leukemias such as acute myelocytic leukemia (AML).

In another embodiment, the present invention relates to a method of treating a patient suffering with cancer comprising administering to the patient Compound A in an amount of from about 400 to about 3200 mg/day, or from about 400 to about 1600 mg/day, or from about 1000 to about 2500 mg/day, or from about 1250 to about 1800 mg/day, for an administration period of up to about 7 days, preferably once per week or up to about 5 days, more preferably once per week, on days 1-3, or on days 1-5, of a 28 day treatment cycle, followed by a rest period of from about 21 to about 23 days, preferably up to about 23 days together with, in combination with Compound A, capecitabine in an amount of about 800-1500 mg/$m^2$ twice daily over a period of 14 days.

The course of a preferred treatment cycle is about 28 days, though cycles anywhere between about 14 and about 28 days are contemplated. This treatment cycle is repeated for as long as the tumor remains under control and the regimen is clinically tolerated.

Dosages of Compound A can be applied either as a body surface area ("BSA") adapted dose (mg/$m^2$/day) or following flat dosing (mg/day). Compound A may be administered as a single dose daily or divided into multiple daily doses.

A patient's body measurement in square meters ("$m^2$") typically ranges from about 1.4 $m^2$ to about 2.2 $m^2$. Thus, the total amount of Compound A to be delivered in a treatment cycle (mg) using a BSA adapted dose would be calculated as follows:

$$[\text{Dose intensity (mg/m}^2\text{/week)}] \times [\text{BSA(m}^2\text{)}] \times [\text{number of weeks in treatment cycle}].$$

For capecitabine in combination with Compound A the preferred dose is 800-1500 mg/$m^2$ twice daily over a period of 14 days.

In an embodiment, Compound A is administered daily for about 5 days, on days 1-5 of a weekly treatment cycle, followed by a rest period of 23 days ("5+/23-"). Compound A is administered daily, either once or twice (bid) daily, preferably once daily. The compound is administered to the patient in an oral unit dosage form, most preferably in tablet form.

Preferably, the 5 day per week treatment schedule is repeated every twenty-eight days, or as soon as permitted by recovery from toxicity, for so long as the tumor is under control or regressing and the patient tolerates the regimen. Preferably, these treatment cycles are repeated for a total of up to about 12 cycles.

In an embodiment, Compound A is administered daily in an amount from about 400 to about 3000 mg/day for up to about 3 days on days 1-5 of a weekly 28 day cycle.

In an embodiment, Compound A is administered daily in an amount from about 400 to about 1500 mg/day for up to about 5 days on days 1-5 of a weekly 28 day cycle.

In an embodiment, Compound A is administered daily in an amount from about 800 to about 3000 mg/day for up to about 5 days on days 1-5 of a weekly 28 day cycle.

In another embodiment, Compound A is administered daily in an amount from about 800 to about 3200 mg/day weekly on days 1, 7, 15 of a weekly 28 day cycle.

In another embodiment, Compound A is administered daily in an amount from about 1250 to about 1800 mg/day weekly on days 1, 7, 15 of a weekly 28 day cycle.

In an embodiment, Compound A is administered daily in an amount from about 400 to about 1600 mg/day for up to about 7 days on days 1-7 of a weekly 28 day cycle.

In an embodiment, Compound A is administered in an amount from about 400 mg/day to about 1600 mg/day, daily, for up to about 7 days, followed by a rest period of up to about 21 days, said administration starting on the first day of a 28 day treatment cycle.

In an embodiment, Compound A is administered in an amount from about 400 mg/day to about 3200 mg/day, daily, for up to about 3 days, followed by a rest period of up to about 23 days, said administration starting on the first day of a 28 day treatment cycle.

In an embodiment, Compound A is administered in an amount from about 800 mg/day to about 3000 mg/day once per week, followed by a rest period of up to about 23 days, said administration starting on the first day of a 28 day treatment cycle.

In an embodiment, Compound A is administered from about 400 mg/day to about 1500 mg/day.

In an embodiment, Compound A is administered from about 1000 mg/day to about 2500 mg/day.

In an embodiment, Compound A is administered from about 1250 mg/day to about 1800 mg/day.

In an embodiment, Compound A and capecitabine are administered, simultaneously or sequentially, according to treatment groups 6, 7, 8 or 9 according to Example 1.

In an embodiment there is provided a kit comprising: (a) a first component containing one or more oral unit dosage forms of an active ingredient comprising capecitabine and (b) a second component comprising 4-{[(2R,3S,4R,5S)-4-(4-Chloro-2-fluoro- phenyl)-3-(3-chloro-2-fluorophenyl)-4-cyano-5-(2,2-dimethyl-propyl)-pyrrolidine-2-carbonyl]-amino}-3-methoxy-benzoic acid. Within this embodiment, preferably the first component contains a sufficient number of units so that a patient can administer about 800 -1500 mg/$m^2$ of capecitabine twice daily over a period of 14 days and the second component contains a sufficient number of doses so that a patient can administer about 400-3000 mg per day of 4-{ [(2R,3S,4R,5S)-4-(4-Chloro-2-fluorophenyl)-3-(3-chloro-2-fluoro-phenyl)-4-cyano-5-(2,2-dimethyl-propyl)-pyrrolidine-2- carbonyl]-amino }-3-methoxy-benzoic acid for a period of up to 5 days.

Abbreviations used herein are as follows:

| | |
|---|---|
| x | times |
| po | orally |
| bid | twice daily |
| wk | week |
| qd | once daily |
| qdx5 | once daily for five days qweekly or |
| 1x/wk | once a week |
| BWL | body weight loss |
| SD | standard deviation |

The LoVo cell line was selected for implantation in mice as it is a p53 wildtype cell line that lacks MDM2 amplification or overexpression and therefore is felt to be more reflective of clinical reality of the colorectal cancer patient population of interest.

The present invention may be exemplified by controlled preclinical animal studies as shown in the Examples below, which illustrates the invention without limitation.

## EXAMPLES

### Example 1

Compound A and the capecitabine formulations were as follows. If not explicitly indicated otherwise, the amounts provided below are concentrations [mg/ml]. Compound A is used at concentrations of 10 mg/ml and 12.5 mg/ml. Capecitabine is used at concentrations of 12.5 mg/ml, 25mg/ml and 50mg/ml. The vehicle solutions for compound A and capecitabine are as follows:

Vehicle solution for Compound A

Klucel LF: 20.0 mg/mL
Tween 80: 1.0 mg/mL
Methylparaben: 0.9 mg/mL
Propylparaben: 0.1 mg/mL
Water for Injection: Qs to 1.0ml

Vehicle solution for oral suspension of capecitabine

Klucel LF: 20.0 mg/mL
Polysorbate 80: 1.0 mg/mL
Methylparaben: 0.9 mg/mL
Propylparaben: 0.1 mg/mL
Purified Water: Qs to 1.0 ml

Compound A is provided as powder for constitution prior to dosing. The powder can be kept at room temperature. The vehicle solution is prepared immediately prior to constitution or, if prepared earlier, kept at 2-8 °C.

Constitution instructions:

1. Take out the vehicle from the storage refrigerator.
2. Take one vial of the Compound A Powder for Constitution. Add the amount of vehicle indicated on the label. For easier constitution, it is recommended to add a small portion to wet the powder first and then add the remaining amount of vehicle. Mix the suspension using a magnetic bar at low speed for 30 minutes or vortex it till the powder is fully wet and suspended before dosing. If needed, use a spatula to help wet the powder periodically during the course of mixing.
3. Continue mixing while withdraw for dosing.

The capecitabine suspension should be stored at 2-8 °C after preparation. While preparing this suspension good mixing (stir for at least 30 min) is required. Stirring should be continued while dosing the suspension.

The following treatment groups were used in the study

1. Vehicle Control qd x 5 po + qd x 14 po
2. Compound A 80 mg/kg qd x 5 po
3. Compound A 100 mg/kg qweekly x 3 po
4. Capecitabine 200 mg/kg qd x 14 po
5. Capecitabine 400 mg/kg qd x 14 po
6. Compound A 80 mg/kg qd x 5 + Capecitabine 200 mg/kg qd x 14
7. Compound A 80 mg/kg qd x 5 + Capecitabine 400 mg/kg qd x 14
8. Compound A 100 mg/kg qweekly x 3 + Capecitabine 200 mg/kg qd x 14
9. Compound A 100 mg/kg qweekly x 3 + Capecitabine 400 mg/kg qd x 14

**Table 1.: Efficacy Summary**

| Group | Schedule | Mean Tumor Volume | %T/C (EOS) | %Inhibition (EOS) | %Increased Life Span |
|---|---|---|---|---|---|
| Vehicle | qdx5<br>qdx14 | 131.10 | ------- | --------- | ----- |

(continued)

| | | | | | |
|---|---|---|---|---|---|
| Cpd. A | | | | | |
| 80 mg/kg | qdx5 | 130.39 | 38 | 62 | 23 |
| 100mg/kg | qwkly×3 | 130.11 | 45 | 55 | 17 |
| Capecitabine | | | | | |
| 200mg/kg | qdx14 | 130.14 | 28 | 72 | 40 |
| 400mg/kg | qdx14 | 130.66 | 12 | 88 | 49 |
| Cpd A + Capecitabine | | | | | |
| 80mg/kg + 200mg/kg | qdx5,qdx14 | 130.78 | 13 | 87 | 40 |
| 80mg/kg+ 400 mg/kg | qdx5,qdx14 | 131.16 | 5 | 95 | 49 |
| 100mg/kg+ 200mg/kg | qwkly3,qdx14 | 130.13 | 6 | 94 | 77 |
| 100mg/kg+ 400mg/kg | qwkly3,qdx14 | 130.88 | -1 | regression | 109 |

All dosing above is per os.

Tumor Growth Inhibition (TGI) and Assessment of Survival/increase in Life Span (ILS)

Efficacy data was graphically represented as the mean tumor volume ± standard error of the mean (SEM). In addition, tumor volumes of treated groups were presented as percentages of tumor volumes of the control groups (%T/C), using the formula: 100 x $((T - T_0)/(C - C_0))$, where T represented mean tumor volume of a treated group on a specific day during the experiment, $T_0$ represented mean tumor volume of the same treated group on the first day of treatment; C represented mean tumor volume of a control group on the specific day during the experiment, and $C_0$ represented mean tumor volume of the same treated group on the first day of treatment.

Tumor volume (in cubic millimeters) was calculated using the ellipsoid formula: $(D \times (d^2))/2$, where "D" represents the large diameter of the tumor and "d" represents the small diameter. In some cases, tumor regression and/or percent change in tumor volume was calculated using the formula: $((T- T_0)/ T_0) \times 100$, where 'T' represents mean tumor volume of the treated group at a particular day, and '$T_0$' represents mean tumor volume of the same treated group at initiation of treatment.

Statistical analysis was determined by the rank sum test and One Way Anova and a post-hoc Bonferroni t-test (SigmaStat, version 2.0, Jandel Scientific, San Francisco, CA, USA). Differences between groups were considered to be significant when the probability value (p) was ≤0.05.

For survival assessment, the percent of increased life space (ILS) was calculated as: 100 x [(median survival day of treated group - median survival day of control group)/median survival day of control group]. Median survival was determined utilizing Kaplan Meier survival analysis. Survival in treated groups was statistically compared with the vehicle group and survival comparisons were done between groups using the log-rank test (Graph Pad Prism, La Jolla, CA, USA). Differences between groups were considered significant when the probability value (p) was ≤0.05.

For the 80mg/kg Cpd A + 400mg/kg capecitabine arm there were 2 partial tumor regressions; for the 100mg/kg Cpd A + 200 mg/kg capecitabine arm 2 partial tumor regressions and for the 100 mg/kg Cpd A + 400 mg/kg capecitabine arm 6 partial tumor regressions and one total tumor regressions.

Discussion of Results

In the above study Compound A gave monotherapy antitumor activity and survival of 62% (p<0.001), 23% (p<0.0001) and 55% (p<0.001), 17% (p<0.0001) for the 80 mg/kg qdx5 and the 100 mg/kg qweekly regimens, respectively. Capecitabine had single agent antitumor activity [(TGI and survival (ILS)] with 72% (p<0.001), 40% (p<0.0001) and 88% (p<0.001), 49% (p<0.0001) for 200 mg/kg qd and 400 mg/kg qd regimens, respectively.

In combination, the Compound A at 80 mg/kg qdx5 plus capecitabine 200 mg/kg qd gave 87% TGI (p<0.001) and 40% ILS (p<0.0001) results. Compound A in combination with capecitabine 400 mg/kg qd gave 95% with 2/10 partial regressions (p<0.001) and 49% (p<0.0001) survival was rendered.

Compound A at 100 mg/kg qweekly plus capecitabine 200 mg/kg qd gave 94% TGI with 2/10 partial regressions

(p<0.001) and 77% ILS (p<0.0001). For compound A at 200mg/kgin combination with capecitabine 400mg/kg qd regression predominated (>100%) with 6/10 partial and 1/10 complete regressions (p<0.001) and 109% ILS (p<0.0001) survival

Statistical cross comparisons for the above study show the TGI and ILS for both 80 mg/kg qdx5 Compound A + capecitabine doublets are not significantly better than their correlative capecitabine monotherapy arms. The TGI and ILS for 100 mg/kg qweekly Compound A + capecitabine 200mg/kg doublet are significantly better than both correlative monotherapy arms. The TGI in the 100 mg/kg qweekly Compound A + capecitabine 400mg/kg doublet was not significantly better than the correlative capecitabine monotherapy arm, however, ILS was significantly better than both correlative monotherapy arms.

Although TGI was equivalent in both 100 mg/kg qweekly Compound A + capecitabine doublets, ILS was significantly better in the 100 mg/kg qweekly Compound A + capecitabine 400mg/kg doublet alluding to a more sustained regressive effect for this group.

## Claims

1. A pharmaceutical product comprising a) a first component comprising Compound A; and b) a second component comprising capecitabine for use in the simultaneous or sequential treatment of cancer, in particular solid and/or hematological tumors such as breast, colon, colorectal, lung and pancreatic tumors, sarcoma or leukemias such as acute myelocytic leukemia (AML), wherein Compound A is 4-{[(2R,3S,4R,5S)-4-(4-Chloro-2-fluoro phenyl)-3-(3-chloro-2-fluorophenyl)-4-cyano-5-(2,2-dimethyl-propyl)-pyrrolidine-2- carbonyl]-amino}-3-methoxy-benzoic acid.

2. The product for use according to claim 1 wherein the capecitabine is dosed at about 800 to about 1500 mg/m$^2$ twice daily over a period of 14 days.

3. The product for use according to claim 2 wherein the dosage of 4-{ [(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2- carbonyl]-amino}-3-methoxy-benzoic acid in an amount from about 400 mg/day to about 1600 mg/day, daily, for up to about 7 days, followed by a rest period of up to about 21 days, said administration starting on the first day of a 28 day treatment cycle.

4. The product for use according to claim 2 wherein the dosage of 4-{ [(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2- carbonyl]-amino }-3-methoxy-benzoic acid is in an amount from about 400 mg/day to about 3200 mg/day, daily, for up to about 3 days, followed by a rest period of up to about 23 days, said administration starting on the first day of a 28 day treatment cycle.

5. The product for use according to claim 2 wherein the dosage of 4-{ [(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2- carbonyl]-amino }-3-methoxy-benzoic acid is in an amount from about 800 mg/day to about 3000 mg/day once per week, followed by a rest period of up to about 23 days, said administration starting on the first day of a 28 day treatment cycle.

6. The product for use according to claim 2 wherein 4-{ [(2R,3S,4R,5S)-4-(4-Chloro-2-fluorophenyl)-3-(3-chloro-2-fluoro-phenyl)-4-cyano-5-(2,2-dimethyl-propyl)-pyrrolidine-2-carbonyl]-amino }-3-methoxy-benzoic acid is administered in an amount of from about 400 mg/day to about 1500 mg/day.

7. The product for use according to claim 2 wherein 4-{ [(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2-carbonyl]-amino}-3-methoxy-benzoic acid is administered in an amount of from about 1000 mg/day to about 2500 mg/day.

8. The product for use according to claim 2 wherein 4-{ [(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2- carbonyl]-amino}-3-methoxy-benzoic acid is administered in an amount of from about 1250 mg/day to about 1800 mg/day.

9. The product for use according to claim 3 wherein the treatment cycle is repeated every 28 days for up to about 12 cycles.

10. The product for use according to any one of claims 2 to 8 wherein 4-{ [(2R,3S,4R,5S)-4-(4-Chloro-2-fluoro- phenyl)-3-(3-chloro-2-fluoro-phenyl)-4-cyano-5-(2,2-dimethyl-propyl)-pyrrolidine-2- carbonyl]-amino}-3-methoxy-benzoic acid is administered weekly on days 1, 7, 15 of a weekly 28 day cycle.

11. The product for use according to any one of claims 2 to 8 wherein 4-{ [(2R,3S,4R,5S)-4-(4-Chloro-2-fluoro- phenyl)-3-(3-chloro-2-fluoro-phenyl)-4-cyano-5-(2,2-dimethyl-propyl)-pyrrolidine-2- carbonyl]-amino }-3-methoxy-benzoic acid is administered once daily for 5 days weekly every 28 days.

12. A kit comprising: (a) a first component containing one or more oral unit dosage forms of an active ingredient comprising capecitabine and (b) a second component comprising 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2- carbonyl]-amino }-3-methoxy-benzoic acid.

13. The kit of claim 12, wherein the first component contains a sufficient number of units so that a patient can administer about 800 -1500 mg/$m^2$ of capecitabine twice daily over a period of 14 days and the second component contains a sufficient number of doses so that a patient can administer about 400-3000 mg per day of 4-{[(2R,3S,4R,5S)-4-(4-Chloro-2-fluoro- phenyl)-3-(3-chloro-2-fluoro-phenyl)-4-cyano-5-(2,2-dimethyl-propyl)-pyrrolidine-2-carbonyl]-amino}-3-methoxy-benzoic acid for a period of up to 5 days.

**Patentansprüche**

1. Pharmazeutisches Produkt, umfassend a) eine erste Komponente, umfassend Verbindung A; und b) eine zweite Komponente, umfassend Capecitabin, zur Verwendung bei der gleichzeitigen oder sequenziellen Behandlung von Krebs, insbesondere festen und/oder hämatologischen Tumoren, wie Brust-, Kolon-, Dickdarm-, Lungen- und Pankreastumoren, Sarkomen oder Leukämien, wie akuter myeloblastischer Leukämie (AML), wobei Verbindung A 4-{[(2R,3S,4R,5S)-4-(4-Chlor-2-fluor phenyl)-3-(3-chlor-2-fluor-phenyl)-4-cyano-5-(2,2-dimethylpropyl)-pyrrolidin-2-carbonyl]-amino}-3-methoxybenzoesäure ist.

2. Produkt zur Verwendung nach Anspruch 1, wobei das Capecitabin zweimal täglich zu etwa 800 bis etwa 1500 mg/$m^2$ über einen Zeitraum von 14 Tagen dosiert wird.

3. Produkt zur Verwendung nach Anspruch 2, wobei die Dosierung der 4-{[(2R,3S,4R,5S)-4-(4-Chlor-2-fluor phenyl)-3-(3-chlor-2-fluor-phenyl)-4-cyano-5-(2,2-dimethylpropyl)-pyrrolidin-2-carbonyl]-amino}-3-methoxybenzoesäure täglich in einer Menge von etwa 400 mg/Tag bis etwa 1600 mg/Tag über bis zu etwa 7 Tagen, gefolgt von einer Ruheperiode von bis zu etwa 21 Tagen, wobei die Verabreichung am ersten Tag eines 28-tägigen Behandlungszyklus beginnt.

4. Produkt zur Verwendung nach Anspruch 2, wobei die Dosierung der 4-{[(2R,3S,4R,5S)-4-(4-Chlor-2-fluor phenyl)-3-(3-chlor-2-fluor-phenyl)-4-cyano-5-(2,2-dimethylpropyl)-pyrrolidin-2-carbonyl]-amino}-3-methoxybenzoesäure täglich in einer Menge von etwa 400 mg/Tag bis etwa 3200 mg/Tag über bis zu etwa 3 Tagen, gefolgt von einer Ruheperiode von bis zu etwa 23 Tagen erfolgt, wobei die Verabreichung am ersten Tag eines 28-tägigen Behandlungszyklus beginnt.

5. Produkt zur Verwendung nach Anspruch 2, wobei die Dosierung der 4-{[(2R,3S,4R,5S)-4-(4-Chlor-2-fluor phenyl)-3-(3-chlor-2-fluor-phenyl)-4-cyano-5-(2,2-dimethylpropyl)-pyrrolidin-2-carbonyl]-amino}-3-methoxybenzoesäure einmal pro Woche in einer Menge von etwa 800 mg/Tag bis etwa 3000 mg/Tag, gefolgt von einer Ruheperiode von bis zu etwa 23 Tagen erfolgt, wobei die Verabreichung am ersten Tag eines 28-tägigen Behandlungszyklus beginnt.

6. Produkt zur Verwendung nach Anspruch 2, wobei die 4-{[(2R,3S,4R,5S)-4-(4-Chlor-2-fluor-phenyl)-3-(3-chlor-2-fluor-phenyl)-4-cyano-5-(2,2-dimethylpropyl)-pyrrolidin-2-carbonyl]-amino}-3-methoxybenzoesäure in einer Menge von etwa 400 mg/Tag bis etwa 1500 mg/Tag verabreicht wird.

7. Produkt zur Verwendung nach Anspruch 2, wobei die 4-{[(2R,3S,4R,5S)-4-(4-Chlor-2-fluor phenyl)-3-(3-chlor-2-fluor-phenyl)-4-cyano-5-(2,2-dimethylpropyl)-pyrrolidin-2-carbonyl]-amino}-3-methoxybenzoesäure in einer Menge von etwa 1000 mg/Tag bis etwa 2500 mg/Tag verabreicht wird.

8. Produkt zur Verwendung nach Anspruch 2, wobei die 4-{[(2R,3S,4R,5S)-4-(4-Chlor-2-fluor phenyl)-3-(3-chlor-2-fluor-phenyl)-4-cyano-5-(2,2-dimethylpropyl)-pyrrolidin-2-carbonyl]-amino}-3-methoxybenzoesäure in einer Menge von etwa 1250 mg/Tag bis etwa 1800 mg/Tag verabreicht wird.

9. Produkt zur Verwendung nach Anspruch 3, wobei der Behandlungszyklus alle 28 Tage über bis zu etwa 12 Zyklen wiederholt wird.

10. Produkt zur Verwendung nach einem der Ansprüche 2 bis 8, wobei die 4-{[(2R,3S,4R,5S)-4-(4-Chlor-2-fluor-phenyl)-3-(3-chlor-2-fluor-phenyl)-4-cyano-5-(2,2-dimethylpropyl)-pyrrolidin-2-carbonyl]-amino}-3-methoxybenzoesäure wöchentlich an den Tagen 1, 7, 15 eines wöchentlichen 28-tägigen Zyklus verabreicht wird.

11. Produkt zur Verwendung nach einem der Ansprüche 2 bis 8, wobei die 4-{[(2R,3S,4R,5S)-4-(4-Chlor-2-fluor-phenyl)-3-(3-chlor-2-fluor-phenyl)-4-cyano-5-(2,2-dimethylpropyl)-pyrrolidin-2-carbonyl]-amino}-3-methoxybenzoesäure alle 28 Tage einmal täglich über 5 Tage wöchentlich verabreicht wird.

12. Kit umfassend: (a) eine erste Komponente, die eine oder mehrere orale Einheitsdosierungsformen eines Wirkstoffs, umfassend Capecitabin, enthält, und (b) eine zweite Komponente, umfassend 4-{[(2R,3S,4R,5S)-4-(4-Chlor-2-fluor-phenyl)-3-(3-chlor-2-fluor-phenyl)-4-cyano-5-(2,2-dimethylpropyl)-pyrrolidin-2-carbonyl]-amino}-3-methoxybenzoesäure.

13. Kit nach Anspruch 12, wobei die erste Komponente eine ausreichende Anzahl Einheiten enthält, um einem Patienten etwa 800-1500 mg/$m^2$ Capecitabin zweimal täglich über einen Zeitraum von 14 Tagen verabreichen zu können, und die zweite Komponente eine ausreichende Anzahl Dosen enthält, um einem Patienten etwa 400-3000 mg pro Tag der 4-{[(2R,3S,4R,5S)-4-(4-Chlor-2-fluor-phenyl)-3-(3-chlor-2-fluor-phenyl)-4-cyano-5-(2,2-dimethylpropyl)-pyrrolidin-2-carbonyl]-amino}-3-methoxybenzoesäure über einen Zeitraum von bis zu 5 Tagen verabreichen zu können.

**Revendications**

1. Produit pharmaceutique comprenant a) un premier constituant comprenant le composé A ; et b) un second constituant comprenant de la capécitabine pour une utilisation dans le traitement simultané ou séquentiel du cancer, en particulier de tumeurs solides et/ou hématologiques telles que les tumeurs du sein, du côlon, colorectales, du poumon et du pancréas, les sarcomes ou les leucémies telles que la leucémie aiguë myéloïde (LAM), dans lequel le composé A est l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque.

2. Produit pour une utilisation selon la revendication 1, dans lequel la capécitabine est administrée à une dose d'environ 800 à environ 1 500 mg/$m^2$ deux fois par jour sur une période de 14 jours.

3. Produit pour une utilisation selon la revendication 2, dans lequel la dose d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque en une quantité quotidienne d'environ 400 mg/jour à environ 1 600 mg/jour, pendant jusqu'à environ 7 jours, suivie d'une période de repos allant jusqu'à environ 21 jours, ladite administration commençant le premier jour d'un cycle de traitement de 28 jours.

4. Produit pour une utilisation selon la revendication 2, dans lequel la dose d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque consiste en une quantité quotidienne d'environ 400 mg/jour à environ 3 200 mg/jour, pendant jusqu'à environ 3 jours, suivie d'une période de repos allant jusqu'à environ 23 jours, ladite administration commençant le premier jour d'un cycle de traitement de 28 jours.

5. Produit pour une utilisation selon la revendication 2, dans lequel la dose d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque consiste en une quantité une fois par semaine d'environ 800 mg/jour à environ 3 000 mg/jour, suivie d'une période de repos allant jusqu'à environ 23 jours, ladite administration commençant le premier jour d'un cycle de traitement de 28 jours.

6. Produit pour une utilisation selon la revendication 2, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré en une quantité d'environ 400 mg/jour à environ 1 500 mg/jour.

7. Produit pour une utilisation selon la revendication 2, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré en une quantité d'environ 1 000 mg/jour à environ 2 500 mg/jour.

**8.** Produit pour une utilisation selon la revendication 2, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré en une quantité d'environ 1 250 mg/jour à environ 1 800 mg/jour.

**9.** Produit pour une utilisation selon la revendication 3, dans lequel le cycle de traitement est répété tous les 28 jours pendant jusqu'à environ 12 cycles.

**10.** Produit pour une utilisation selon l'une quelconque des revendications 2 à 8, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré de manière hebdomadaire aux jours 1, 7, 15 d'un cycle hebdomadaire de 28 jours.

**11.** Produit pour une utilisation selon l'une quelconque des revendications 2 à 8, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré une fois par jour pendant 5 jours par semaine tous les 28 jours.

**12.** Kit comprenant : (a) un premier constituant contenant une ou plusieurs formes posologiques unitaires orales d'un principe actif comprenant de la capécitabine et (b) un second constituant comprenant l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque.

**13.** Kit selon la revendication 12, dans lequel le premier constituant contient un nombre suffisant d'unités de sorte qu'un patient peut se voir administrer environ 800 à 1 500 $mg/m^2$ de capécitabine deux fois par jour sur une période de 14 jours et le second constituant contient un nombre suffisant de doses de sorte qu'un patient peut se voir administrer environ 400 à 3 000 mg par jour d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque pendant une période allant jusqu'à 5 jours.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4966891 A
- US 5472949 A
- US 5453497 A
- US 5476932 A
- US 53226603 P
- WO 2011098398 A
- US 8354444 B
- EP 2014050974 W
- WO 2008034039 A


### Non-patent literature cited in the description


- *Curr. Pharm. Design,* 2011, vol. 17 (6), 640-652
- *Tumor Biol.,* 2010, vol. 31, 287-295